# FASCICULE DE BREVET EUROPEEN

(11) **EP 1 343 458 B1**
(45) Date de publication et mention de la délivrance du brevet: **13.02.2008**
(21) Numéro de dépôt: 01270307.0
(22) Date de dépôt: 11.12.2001
(51) Int. Cl.: A61K 8/04, A61K 8/30, A61K 8/72, A61K 8/92, A61K 8/00, A61K 8/96, A61Q 1/00, A61Q 1/02, A61Q 1/12, A61Q 19/00, A61Q 3/00, A61Q 3/02, A61Q 5/00, C08L 101/00

(54) **COMPOSITION COSMETIQUE COMPRENANT UN MELANGE DE POLYMERES**
KOSMETISCHE ZUSAMMENSETZUNG MIT EINEM POLYMERGEMISCH
COSMETIC COMPOSITION COMPRISING A MIXTURE OF POLYMERS

(30) Priorité: 12.12.2000 FR 0016161
(43) Date de publication de la demande: 17.09.2003
(73) Titulaire: L'ORÉAL, 75008 Paris (FR)
(72) Inventeur: COLLIN, Nathalie, F-92330 Sceaux (FR)
(74) Mandataire: Boulard, Denis
(86) Numéro de dépôt international: PCT/FR2001/003940
(87) Numéro de publication internationale: WO 2002/047630

(56) Documents cités:
- EP-A- 0 557 196
- EP-A- 0 749 746
- EP-A- 0 749 747
- EP-A- 0 847 752
- EP-A- 0 923 928
- EP-A- 0 930 060
- EP-A- 1 048 282
- WO-A-95/15741
- FR-A- 2 785 179
- US-A- 3 148 125
- US-A- 3 645 705
- US-A- 5 500 209
- US-A- 5 783 657
- US-A- 5 998 570

## Description

La présente invention se rapporte à une composition comprenant, dans un milieu physiologiquement acceptable, une dispersion de particules de polymère filmogène et un polymère à hétéroatome spécifique, destinée en particulier au domaine cosmétique. L'invention se rapporte également à un procédé de maquillage ou de soin cosmétiques des matières kératiniques. La composition et le procédé de maquillage ou de soin selon l'invention sont plus particulièrement destinés aux matières kératiniques d'êtres humains telles que la peau (y compris du cuir chevelu), les ongles, les fibres kératiniques, notamment sensiblement longitudinales, telles que les cils, les sourcils et les cheveux. Plus spécialement, l'invention porte sur un mascara.

La composition selon l'invention peut se présenter sous la forme de composition de revêtement des cils (notamment de mascara), d'eye-liner, de produit pour les sourcils, de produit pour les lèvres, de fard à joues ou à paupières, de fond de teint, de produit de maquillage du corps, de produit anti-cernes, de vernis à ongles, de produit de soin de la peau, y compris du cuir chevelu, de produit pour les cheveux (mascara pour cheveux, spray).

La composition de maquillage peut également être appliquée sur les accessoires de maquillage (support) comme les, faux cils, postiches, perruques, faux ongles ou encore sur des pastilles ou patchs adhérents sur la peau ou les lèvres (du type mouches).

Les compositions de revêtement des cils, appelées mascara, comprennent généralement, de façon connue, au moins une cire sous la forme d'émulsion cire-dans-eau et au moins un polymère filmogène pour déposer un film de maquillage sur les cils et gainer ces derniers, comme le décrit par exemple le document WO-A-95/15741. Les utilisatrices attendent pour ces produits de bonnes propriétés cosmétiques telles que l'adhérence sur les cils, un allongement ou un recourbement des cils, ou bien encore une bonne tenue du mascara dans le temps, notamment une bonne résistance aux frottements par exemple des doigts ou des tissus (mouchoirs, serviettes).

Toutefois, avec ces compositions, les propriétés de maquillage comme le gainage, l'allongement ou le recourbement des cils sont obtenus lorsqu'une quantité importante de produit est déposée sur les cils à l'aide d'un applicateur, telle qu'une brosse à mascara. Lorsque la composition n'adhère pas bien sur les cils, l'utilisatrice doit appliquer plusieurs fois la brosse imprégnée de produit sur les cils, ce qui demande de consacrer un certain temps pour se maquiller et obtenir les résultats de maquillage souhaités. Or ce temps peut être perçu comme beaucoup trop long par les utilisatrices pressées. Un besoin existe donc de disposer de mascaras permettant d'obtenir rapidement et facilement le maquillage attendu.

Le but de la présente invention est de disposer d'une composition de maquillage des matières kératiniques, notamment des fibres kératiniques tels que les cils, s'appliquant facilement sur les matières kératiniques et conduisant rapidement à un maquillage présentant de bonnes propriétés cosmétiques.

Les inventeurs ont constaté de façon surprenante que l'utilisation d'un polymère à hétéroatome particulier dans une composition comprenant des particules d'un polymère filmogène dispersées dans le milieu de la composition permet d'améliorer les propriétés d'adhérence de la composition sur les matières kératiniques, notamment sur les fibres kératiniques comme les cils. La composition s'applique facilement sur les matières kératiniques et permet de déposer rapidement la composition en quantité suffisante pour obtenir un maquillage présentant les propriétés cosmétiques attendues. En particulier, on obtient rapidement un dépôt épais du maquillage sur les matières kératiniques ce qui évite aux utilisatrices d'appliquer trop longtemps la composition sur les matières kératiniques.
Ainsi, pour un mascara, on obtient un maquillage qui épaissit rapidement les fibres kératiniques, notamment les cils ; on constate ainsi une charge instantanée des cils. De plus, le mascara confère un bon allongement aux cils maquillés.

De façon plus précise, l'invention a pour objet une composition comprenant, dans un milieu physiologiquement acceptable, au moins un premier polymère de masse moléculaire moyenne en poids inférieure à 100 000, comportant a) un squelette polymérique, ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, et une dispersion de particules d'un deuxième polymère filmogène, insoluble dans ledit milieu.

L'invention a également pour objet un procédé cosmétique de maquillage ou de soin des matières kératiniques des êtres humains comprenant l'application sur les matières kératiniques d'une composition telle que définie précédemment. De préférence, le procédé s'applique aux fibres kératiniques seniblement longitudinales, comme les cils, les cheveux, les sourcils) et plus spécialement aux cils.

L'invention a encore pour objet l'utilisation d'une composition telle que définie précédemment pour l'obtention d'un dépôt adhérent et/ou d'un maquillage rapide sur les matières kératiniques.

L'invention a pour autre objet l'utilisation d'un mascara comprenant une composition telle que définie précédemment pour épaissir rapidement et/ou allonger les cils.

L'invention a également pour objet l'utilisation, dans une composition physiologiquement acceptable, de l'association d'au moins un premier polymère de masse moléculaire moyenne en poids inférieure à 100 000 et mieux inférieure à 50 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés, et d'au moins une dispersion de particules un deuxième polymère filmogène insoluble dans ledit milieu, pour l'obtention d'un dépôt adhérent sur les matières kératiniques et/ou d'un maquillage rapide des matières kératiniques et/ou pour épaissir rapidement et/ou allonger les cils.

Par milieu physiologiquement acceptable, on entend un milieu non toxique et susceptible d'être appliquée sur la peau, les phanères ou les lèvres d'êtres humains, comme un milieu cosmétique.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substituées au moins partiellement par des atomes de fluor.

Selon l'invention, ces chaînes peuvent être liées directement au squelette polymérique ou via une fonction ester ou un groupement perfluoré.

Par "polymère", on entend au sens de l'invention un composé ayant au moins 2 motifs de répétition, et de préférence au moins 3 motifs de répétition.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes avantageusement non pendants et se trouvant dans le squelette polymérique. Ces hétéroatomes sont choisis parmi les atomes d'azote, de soufre, de phosphore et leurs associations, associés éventuellement à un ou plusieurs atome d'oxygène. De préférence, les motifs comportent au moins un atome d'azote en particulier non pendant. Ces motifs comportent, en outre, avantageusement, un groupe carbonyle.

Les motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide. Avantageusement, les chaînes pendantes sont liées directement à l'un au moins des hétéroatomes du squelette polymérique.

Le premier polymère peut comprendre entre les motifs hydrocarbonés des motifs siliconés ou des motifs oxyalkylénés.

En outre, le premier polymère de la composition de l'invention comprend avantageusement de 40 à 98 % de chaînes grasses par rapport au nombre total des motifs à hétéroatome et des chaînes grasses et mieux de 50 à 95 %. La nature et la proportion des motifs à hétéroatome est fonction de la nature de la phase grasse et est en particulier similaire à la nature polaire de la phase grasse. Ainsi, plus les motifs à hétéroatome sont polaires et en proportion élevée dans le premier polymère, ce qui correspond à la présence de plusieurs hétéroatomes, plus le premier polymère a de l'affinité avec les huiles polaires. En revanche, plus les motifs à hétéroatome sont peu polaires voire apolaires ou en proportion faible, plus le premier polymère a de l'affinité avec les huiles apolaires.

Le premier polymère est avantageusement un polyamide. Aussi, l'invention a également pour objet une composition contenant, dans un milieu cosmétiquement acceptable, au moins un premier polymère de polyamide de masse moléculaire moyenne en poids inférieure à 100 000, comportant a) un squelette polymérique, ayant des motifs répétitifs amide, et b) éventuellement au moins une chaîne grasse pendante et/ou au moins une chaîne terminale éventuellement fonctionnalisées, ayant de 8 à 120 atomes de carbone et étant liées à ces motifs amide, et une dispersion de particules d'un deuxième polymère filmogène insoluble dans ledit milieu.

De préférence, lés chaînes grasses pendantes sont liées à l'un au moins des atomes d'azote des motifs amide du premier polymère.

En particulier, les chaînes grasses de ce polyamide représentent de 40 à 98 % du nombre total des motifs amide et des chaînes grasses, et mieux de 50 à 95 %.

Avantageusement, le premier polymère, et en particulier le polyamide, de la composition selon l'invention présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000 ,et mieux de 2000 à 10 000.

Le premier polymère, et en particulier le polyamide, est non soluble dans l'eau, notamment à 25 °C. En particulier, il ne comporte pas de groupe ionique.

Comme premiers polymères préférés utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone, chaque chaîne grasse terminale étant liée au squelette polyamide par au moins un groupe de liaison en particulier ester. De préférence, ces polymères comportent une chaîne grasse à chaque extrémité du squelette polymérique et en particulier du squelette polyamide. Comme autre groupe de liaison on peut citer les groupes éther, amine, urée, uréthane, thioester, thiurée, thiouréthane.

Ces premiers polymères sont de préférence des polymères résultant d'une polycondensation entre un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone. Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5783657 de la société Union Camp. Chacun de ces polymères satisfait notamment à la formule (1) suivante : 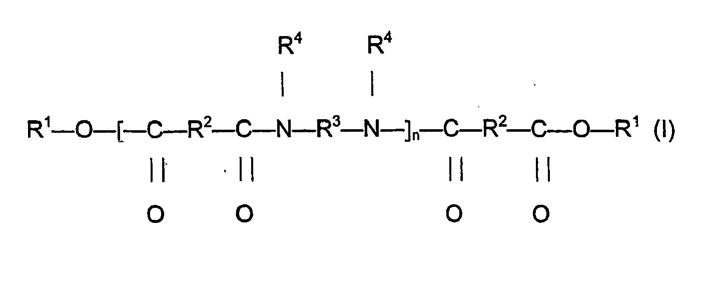 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

Dans le cas particulier de la formule (I), les chaînes grasses terminales éventuellement fonctionnalisées au sens de l'invention sont des chaînes terminales liées au dernier hétéroatome, ici l'azote, du squelette polyamide.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5 et mieux supérieur à 2. De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux au moins 75 % des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (I) se présentent sous forme de mélanges de polymères, ces mélanges pouvant en outre contenir un produit de synthèse correspondant à un composé de formule (I) où n vaut 0, c'est-à-dire un diester.

A titre d'exemple de premiers polymères selon l'invention, on peut citer les produits commerciaux vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymères d'un diacide en C₃₆ condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

Comme premier polymère utilisable dans l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathie-son Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Il est aussi possible d'utiliser des résines de polyamides issues de légumes comme celles décrites dans les brevets US-A-5783657 et US-A-5998570.

Le premier polymère présent dans la composition selon l'invention a avantageusement une température de ramollissement supérieure à 65°C et pouvant aller jusqu'à 190°C. De préférence, il présente une température de ramollissement allant de 70 à 130°C et mieux de 80 à 105°C. Le premier polymère est en particulier un polymère non cireux.

De préférence, le premier polymère selon l'invention répond à la formule (I) mentionnée précédemment. Ce premier polymère présentent du fait de leur(s) chaîne(s) grasse(s), une bonne solubilité dans les huiles et donc conduisent à des compositions macroscopiquement homogènes même avec un taux élevé (au moins 25%) de polymère, contrairement à des polymères exempts de chaîne grasse.

Le premier polymère peut être présent dans la composition selon l'invention en une teneur allant de 0,01 % à 10 % en poids, par rapport au poids total de la composition, de préférence allant de 0,05 % à 5 % en poids, et mieux allant de 0,1 % à 3 % en poids.

La composition selon l'invention peut comprendre une phase grasse pouvant comprendre des corps gras choisis parmi les huiles, les solvants organiques, les cires, les corps gras pâteux, et leurs mélanges. La phase grasse peut former une phase continue de la composition. En particulier, la composition selon l'invention peut être anhydre.

La phase grasse peut notamment être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange, dans la mesure où elles forment un mélange homogène et stable et où elles sont compatibles avec l'utilisation envisagée.

La phase grasse totale de la composition, qui peut être une phase grasse liquide, peut représenter de 2 % à 98 % en poids, par rapport au poids total de la composition, et de préférence de 5 à 85 % en poids.

Avantageusement, la phase grasse de la composition peut comprendre au moins une huile ou solvant organique volatile et/ou au moins une huile non volatile.

Par "huile ou solvant organique volatile", on entend au sens de l'invention tout milieu non aqueux susceptible de s'évaporer au contact de la peau en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 10⁻² à 300 mm de Hg (0,13 Pa à 40.000 Pa) et de préférence supérieure à 0,3 mm de Hg (30 Pa). Par "huile non volatile", on entend une huile restant sur la peau à température ambiante et pression atmosphérique au moins plusieurs heures et ayant notamment une pression de vapeur inférieure à 10⁻² mm de Hg (1,33 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C₈-C₁₆ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutàne ou le perfluorométhylcyclopentane.

L'huile volatile peut être présente dans la composition selon l'invention en une teneur allant de 0 % à 98 % en poids (notamment de 0,1 % à 98 %), par rapport au poids total de la composition, de préférence de 0 % à 65 % en poids (notamment de 1 % à 65 %).

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₅ + R₆ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0 à 80 % (notamment de 0,1 à 80 %) en poids, de préférence de 0 % à 50 % en poids (notamment 0,1 à 50 %), par rapport au poids total de la composition, et mieux de 0 % à 20 % en poids (notamment 0,1 % à 20 %).

La composition selon l'invention peut également comprendre une cire. Par "cire", on entend au sens de la présente invention, un composé gras lipophile, solide à température ambiante (25°C) et pression atmosphérique (760 mm de Hg, soit 10⁵ Pa), à changement d'état solide/liquide réversible, ayant une température de fusion supérieure à 30°C et mieux supérieure à 55 °C et pouvant aller jusqu'à 200° C, notamment jusqu'à 120 °C.
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10°C par minute.

Les cires, au sens de l'invention, sont celles généralement utilisées dans les domaines cosmétique et dermatologique. On peut notamment citer la cire d'abeilles, la cire de lanoline, et les cires d'insectes de Chine; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et mieux à plus de 55°C.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone ou les cires fluorées.

Les cires présentes dans la composition peuvent être dispersées sous forme de particules dans une phase aqueuse telle que définie ci-après. Ces particules peuvent avoir une taille moyenne allant de 50 nm à 10 µm, et de préférence de 50 nm à 3,5 µm.
En particulier, la cire peut être présente sous forme d'émulsion cires-dans-eau, les cires pouvant être sous forme de particules de taille moyenne allant de 1 µm à 10 µm, et de préférence de 1 µm à 3,5 µm.
Dans un autre mode de réalisation de la composition selon l'invention, la cire peut être présente sous forme de microdispersion de cire, la cire étant sous forme de particules dont la taille moyenne est inférieure à 1 µm, et va notamment de 50 nm à 500 nm. Des microdispersions de cires sont décrites dans les documents EP-A-557196, EP-A-1048282.

La cire peut être également présente dans un phase grasse liquide en mélange avec des huiles telles que définies précédemment.

La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20°C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

La cire peut être présente dans la composition selon l'invention en une teneur allant de 0,1 % à 50 % en poids, par rapport au poids total de la composition, de préférence de 0,5 % à 30 % en poids, et mieux de 1% à 20 % en poids.

La composition selon l'invention peut comprendre au moins un composé gras pâteux à température ambiante. Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le "THIXINR" de Rhéox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl di-methicones notamment ceux vendus par la société Dow Corning sous les noms commerciaux de DC2503 et DC25514, et leurs mélanges.

Le corps gras pâteux peut être présent dans la composition selon l'invention en une teneur allant de 0 à 60% (notamment 0,01 % à 60 %) en poids, par rapport au poids total de la composition, de préférence allant de 0,5 à 45 % en poids, et mieux allant de 2 % à 30 % en poids, dans la composition.

La composition selon l'invention peut également comprendre un milieu aqueux, constituant une phase aqueuse, qui peut être la phase continue de la composition.

La phase aqueuse peut être constituée essentiellement d'eau; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.
La phase aqueuse (eau et éventuellement le solvant organique miscible à l'eau) peut être présente, en une teneur allant de 1 % à 95 % en poids, par rapport au poids total de la composition, de préférence de 3 % à 80 % en poids, et mieux de 5 % à 60 % en poids.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention comprend au moins un deuxième polymère filmogène, différent du premier polymère décrit précédemment, sous forme de particules solides dispersées dans le milieu physiologiquement acceptable. Ces particules peuvent être dispersées dans une phase aqueuse ou bien dans une phase grasse liquidé. La composition peut comprendre un mélange de ces polymères. Le deuxième polymère filmogène est insoluble dans le milieu de la composition, c'est à dire qu'il reste à l'état de particules dans le mélange des ingrédients de la composition formant le milieu physiologiquement acceptable. Ainsi, par "polymère insoluble dans le milieu physiologiquement acceptable", il faut entendre un polymère dont la solubilité dans ce milieu est inférieure à 1 % en poids.

Le deuxième polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

On utilise de préférence un polymère filmogène apte à former un film hydrophobe, c'est-à-dire un polymère dont le film a une solubilité dans l'eau à 25 °C inférieure à 1 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Il est possible d'utiliser tout monomère connu de l'homme du métier entrant dans les catégories de monomères acryliques et vinyliques, (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ^{®} par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le deuxième polymère filmogène peut être présent sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®}, NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEY KOGYO; ou bien encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425 ^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

En outre, la phase aqueuse de la composition peut comprendre un polymère additionnel hydrosoluble présent dans le milieux aqueux de la composition sous forme solubilisée. Comme exemples de polymères filmogènes hydrosolubles, on peut citer
- les protéines comme les protéines d'origine végétale telles que les protéines de blé, de soja ; les protéines d'origine animale tels que les kératines, par exemples les hydrolysats de kératine et les kératines sulfoniques;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quatemisés de la cellulose ;
- les polymères ou copolymères acryliques, tels que les polyacrylates ou les poly-méthacrylates;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle; les copolymères de vinylpyrrolidone et de caprolactame ; l'alcool polyvinylique ;
- les polymères d'origine naturelle, éventuellement modifiés, tels que :
   . les gommes arabiques, la gomme de guar, les dérivés du xanthane, la gomme de karaya ;
   . les alginates et les carraghénanes ;
   . les glycoaminoglycanes, l'acide hyaluronique et ses dérivés ;
   . la résine shellac, la gomme de sandaraque, les dammars, les élémis, les copals ;
   . l'acide désoxyribonucléïque ;
   . les muccopolysaccharides tels que l'acide hiraluronique, les chondroïtines sulfate, et leurs mélanges.

Selon une autre variante de réalisation de la composition selon l'invention, le deuxième polymère filmogène peut être présent sous forme de particules, stabilisées en surface, dispersées dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.
De préférence, la phase grasse liquide comprend une huile volatile, éventuellement en mélange avec une huile non volatile, les huiles pouvant être choisies parmi les huiles citées précédemment.

La dispersion de particules de polymère stabilisées en surface peut être fabriquée comme décrit dans le document EP-A-749747. Elle peut être obtenue par polymérisation en dispersion, c'est-à-dire par précipitation du polymère en cours de formation, avec protection des particules formées avec un stabilisant.

Le choix de la phase grasse liquide est effectué par l'homme du métier en fonction de la nature des monomères constituant le polymère et/ou de la nature du stabilisant, comme indiqué ci-après.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

Le stabilisant peut aussi être choisi parmi les copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl(C₂-C₁₈) méthyl siloxane ; le bloc polyéther peut être un poly alkylène en C₂-C₁₈, en particulier polyoxyéthylène et/ou polyoxypropylène. En particulier, on peut utiliser les diméthicones copolyol ou encore les alkyl (C₂-C₁₈) méthicones copolyol. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".

Comme copolymères blocs greffés ou séquencés, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly-(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également employer des copolymères de (méth)acrylates d'alkyl en C₁-C₄, et de (méth)acrylates d'alkyl en C₈-C₃₀. On peut en particulier citer le copolymère méthacrylate de stéaryle/méthacrylate de méthyle.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque la phase grasse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxy (C₂-C₁₈)alkylène et notammment polyoxypropyléné et/ou oxyéthyléné.

Lorsque la phase grasse liquide ne comprend pas d'huile de silicone, l'agent stabilisant est de préférence choisi dans le groupe constitué par :
- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyl en C₁-C₄, et d'acrylates ou de méthacrylates d'alkyl en C₈-C₃₀,
- (c) les copolymères blocs greffés ou séquencés comprenant au moins un bloc résultant de la polymérisation d'au moins un monomère éthylénique, à liaisons éthyléniques conjuguées,
et au moins un bloc d'un polymère vinylique ou acrylique ou d'un polyéther ou d'un polyester, ou leurs mélanges.

De préférence, on utilise des polymères dibloc comme agent stabilisant.

La taille des particules du deuxième polymère filmogène en dispersion soit dans la phase aqueuse, soit dans la phase grasse liquide, peut aller de 5 nm à 600 nm, et de préférence de 20 nm à 300 nm.

La composition selon l'invention peut comprendre un agent auxiliaire de filmification favorisant la formation d'un film avec le deuxième polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

En outre, la phase grasse liquide peut aussi contenir un troisième polymère filmogène additionnel solubilisé dans la phase grasse liquide, dit encore polymère liposoluble.

Comme polymère liposoluble, on peut notamment citer les copolymères résultant de la copolyméisation d'au moins un ester vinylique et d'au moins un autre monomère qui peut être une oléfine, un alkylvinyléther ou un ester allylique ou méthallylique, comme décrits dans la demande FR-A-2232303, dont le contenu est incorporé dans la présente demande à titre de référence.

Comme polymères filmogènes liposolubles utilisables dans l'invention, on peut également citer les polyalkylènes et notamment les copolymères d'alcènes en C₂-C₂₀ comme !e polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène liposoluble peut être présent dans la composition en une teneur allant de 0,1 % à 15 % en poids, par rapport au poids total de la composition, et mieux de 2 % à 10 % en poids.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi, les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet.2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels que les antioxydants, les charges, les conservateurs, les parfums, les neutralisants, les épaississants, les actifs cosmétiques ou dermatologiques comme par exemple des émollients, des hydratants, des vitamines, des filtres solaires, et leurs mélanges. Ces additifs peuvent être présents dans la composition en une teneur allant de 0 à 20% (notamment de 0,01 à 20 %) du poids total de la composition et mieux de 0,01 à 10% (si présents).

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique ou dermatologique.

L'invention est illustrée plus en détail dans les exemples suivants.

### Exemple 1 :

On a préparé un mascara ayant la composition suivante :

| | |
|---|---|
| - Cire de carnauba | 2,6 g |
| - Cire d'abeille | 3,3 g |
| - Cire de paraffine | 10,4 g |
| - Huile de jojoba hydrogénée | 0,2g |
| - Huile de palme hydrogénée | 0,2 g |
| - Résine de polyamide avec groupes ester terminaux vendu sous la dénomination "UNICLEAR^{®} 100" par la société Arizona Chemical | 1 g |
| - Amino-2 méthyl-2 propanediol-1,3 | 0,8 g |
| - Triéthanolamine | 2,4 g |
| - Acide stéarique | 6,6 g |
| - Hydroxyéthylcellulose | 0,8 g |
| - Gomme arabique | 0,6 g |
| - Copolymère acrylate d'éthyle/méthacrylate de méthyle (80/20) en dispersion aqueuse à 50 % MA (DAITOSOL 5000 AD de SAITO) | 7 g MA |
| - Oxyde de fer noir | 5 g |
| - Conservateurs qs | |
| - Eau qsp | 100 g |

Ce mascara s'applique facilement, adhère bien sur les cils pendant et après l'application ; ces derniers sont maquillés rapidement. Il confère également une charge instantannée des cils.

### Exemple 2 :

On a préparé un mascara ayant la composition suivante :

| | |
|---|---|
| - Cire de carnauba | 4,6 g |
| - Cire de son de riz | 2,1 g |
| - Paraffine | 2,2 g |
| - Cire d'abeille | 8,2 g |
| - Résine de polyamide avec groupes ester terminaux vendu sous la dénomination "UNICLEAR^{®} 100" par la société | |
| Arizona Chemical | 1 g |
| Talc | 1 g |
| - Bentonite | 5 g |
| - Copolymère acétate de vinyle/stéarate d'allyle (65/35) | |
| (Mexomère PQ de CHIMEX) | 6,5 g |
| - Polylaurate de vinyle (Mexomère PP de CHIMEX) | 0,7 g |
| - Sulfopolyester (AQ 555 d'EASTMAN CHEMICAL) | 0,12 g |
| - Isododécane | 53,9 g |
| - Carbonate de propylène | 1,6 g |
| - Pigments | 4,9g |
| - Conservateurs qs | |
| - Eau qsp | 100 g |

Ce mascara adhère bien sur les cils pendant et après l'application. Il confère aux cils une bonne charge instantannée.

### Exemple 3 :

### a) Dispersion de polymère dans l'isododécane utilisée :

On a préparé une dispersion de copolymère non réticulé d'acrylate de méthyle et d'acide acrylique dans un rapport 95/5, dans de l'isododécane, selon la méthode de l'exemple 7 du document EP-A-749 747. On obtient ainsi une dispersion de particules de poly(acrylate de méthyle/acide acrylique) stabilisées en surface dans de l'isododécane par un copolymère dibloc séquencé polystyrènelcopoly(éhylène-propylène) vendu sous le nom de KRATON G1701 (Shell), ayant un taux de matière sèche de 24,2% en poids et une taille moyenne des particules de 180 nm et une Tg de 20°C. Ce copolymère est filmifiable à température ambiante.

### b) On a préparé un mascara ayant la compositions suivante:

| | |
|---|---|
| - Cire de carnauba | 4,7 g |
| - Cire de son de riz | 2,1 g |
| - Paraffine | 2,2 g |
| - Cire d'abeille | 8,2 g |
| - Résine de polyamide avec groupes ester terminaux vendu | |
| sous la dénomination "UNICLEAR^{®} 100" par la société | |
| Arizona Chemical | 0,5 g |
| - Dispersion de polymère dans l'isododécane selon a) | 10 g |
| - Talc | 1 g |
| - Bentonite | 5 g |
| - Copolymère acétate de vinyle/stéarate d'allyle (65/35) | |
| (Mexomère PQ de CHIMEX) | 6,5 g |
| - Polylaurate de vinyle (Mexomère PP de CHIMEX) | 0,7 g |
| - Carbonate de propylène | 1,6 g |
| - Pigments | 4,9 g |
| - Conservateurs qs | |
| - Isododécane qsp | 100 g |

Ce mascara adhère bien sur les cils pendant et après l'application. Il confère aux cils une bonne charge instantannée.

## Revendications

1. Composition comprenant, dans un milieu physiologiquement acceptable, au moins un premier polymère de masse moléculaire moyenne en poids inférieure à 100 000, comportant a) un squelette polymérique, ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, et une dispersion de particules d'un deuxième polymère filmogène insoluble dans ledit milieu.

2. Composition selon la revendication 1, **caractérisée par le fait que** la masse molaire moyenne du premier polymère est inférieure à 50 000.

3. Composition selon la revendication 1 ou 2, **caractérisée par le fait que** les motifs à hétéroatome du premier polymère comportent un atome d'azote.

4. Composition selon l'une des revendications précédentes, **caractérisée par le fait que** les motifs à hétéroatome du premier polymère sont des groupes amides.

5. Composition selon l'une des revendications précédentes, **caractérisée par le fait que** les chaînes grasses représentent de 40 à 98 % et mieux de 50 à 95 % du nombre total des motifs à hétéroatome et des chaînes grasses.

6. Composition selon l'une des revendications précédentes, **caractérisée par le fait que** les chaînes grasses pendantes sont liées directement à l'un au moins desdits hétéroatomes.

7. Composition comprenant, dans un milieu physiologiquement acceptable, au moins un premier polymère de polyamide de masse moléculaire moyenne en poids inférieure à 100 000, comportant a) un squelette polymérique ayant des motifs répétitifs amide, et b) éventuellement au moins une chaîne grasse pendante et/ou au moins une chaîne grasse terminale éventuellement fonctionnalisées, ayant de 8 à 120 atomes de carbone et étant liées à ces motifs amide, et une dispersion de particules d'un deuxième polymère filmogène insoluble dans ledit milieu.

8. Composition selon la revendication précédente, **caractérisée par le fait que** les chaînes grasses représentent de 40 à 98 % du nombre total des motifs amide et des chaînes grasses.

9. Composition selon la revendication 7 ou 8, **caractérisée par le fait que** les chaînes grasses représentent de 50 à 95 % du nombre total des motifs amide et des chaînes grasses.

10. Composition selon l'une des revendications 7 à 10, **caractérisée par le fait que** les chaînes grasses pendantes sont liées directement à l'un au moins des atomes d'azote des motifs amide.

11. Composition selon l'une des revendications précédentes, **caractérisée par le fait que** la masse molaire moyenne du premier polymère va de 1000 à 100 000, de préférence de 1000 à 50 000, et mieux de 1 000 à 30 000.

12. Composition selon l'une des revendications précédentes, **caractérisée par le fait que** la masse molaire moyenne en poids du premier polymère filmogène va de 2 000 à 20 000, et de préférence de 2 000 à 10 000.

13. Composition selon l'une des revendications précédentes, **caractérisée par le fait que** la ou les chaînes grasses terminales sont liées au squelette par des groupes de liaison.

14. Composition selon la revendication 13, **caractérisée par le fait que** les groupes de liaison sont des groupes ester.

15. Composition selon l'une des revendications précédentes, **caractérisée par le fait que** la ou les chaînes grasses ont de 12 à 68 atomes de carbone.

16. Composition selon l'une des revendications précédentes, **caractérisée par le fait que** le premier polymère est choisi parmi les polymères de formule (I) suivante et leurs mélanges : 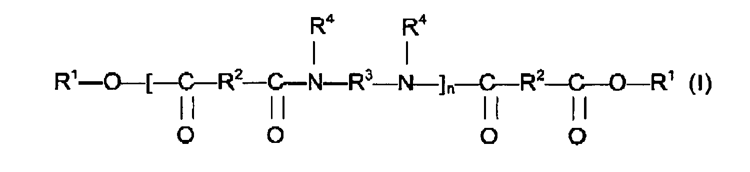 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

17. Composition selon la revendication précédente, **caractérisée par le fait que** R¹ est un groupe alkyle en C₁₂ à C₂₂.

18. Composition selon l'une des revendications 15 ou 16, **caractérisée par le fait que** R² sont des groupes ayant de 30 à 42 atomes de carbone.

19. Composition selon l'une des revendications précédentes, **caractérisée par le fait que** le premier polymère est présent en une teneur allant de 0,01 % à 10 % en poids, par rapport au poids total de la composition, de préférence allant de 0,05 % à 5 % en poids, et mieux allant de 0,1 % à 3 % en poids.

20. Composition selon l'une quelconque des revendications précédentes, **caractérisée par le fait que** le deuxième polymère filmogène est choisi dans le groupe formé par les polymères radicalaires, les polycondensats, les polymères d'origine naturelle et leurs mélanges.

21. Composition selon l'une quelconque des revendications précédentes, **caractérisée par le fait que** le deuxième polymère filmogène est choisi dans le groupe formé par les polymères vinyliques, les polyuréthanes, les polyesters, les polymères cellulosiques.

22. Composition selon l'une quelconque des revendications précédentes, **caractérisée par le fait qu'**elle comprend une phase aqueuse.

23. Composition selon la revendication 22, **caractérisée par le fait que** la phase aqueuse comprend de l'eau et éventuellement un solvant organique miscible à l'eau.

24. Composition selon la revendication 23 , **caractérisée par le fait que** le solvant organique miscible à l'eau est choisi dans le groupe formé par les monoalcools inférieurs ayant de 1 à 5 atomes de carbone, les glycols ayant de 2 à 8 atomes de carbone, les cétones en C₃-C₄, les aldéhydes en C₂-C₄.

25. Composition selon la revendication 23 ou 24, **caractérisée par le fait que** le solvant organique miscible à l'eau est choisi dans le groupe formé par l'éthanol, l'isopropanol, le propylène glycol, l'éthylène glycol, le 1,3-butylène glycol, le dipropylène glycol.

26. Composition selon l'une quelconque des revendications 23 à 25, **caractérisée par le fait qu'**elle comprend de l'eau en une teneur allant de 1 % à 95 % en poids, par rapport au poids total de la composition.

27. Composition selon l'une quelconque des revendications précédentes, **caractérisée par le fait que** le deuxième polymère filmogène est présent sous la forme de particules dispersées dans une phase aqueuse.

28. Composition selon l'une quelconque des revendications 22 à 27, **caractérisée par le fait que** la phase aqueuse comprend un polymère filmogène additionnel hydrosoluble.

29. Composition selon l'une quelconque des revendications précédentes, **caractérisée par le fait qu'**elle comprend une phase grasse liquide.

30. Composition selon la revendication 29, **caractérisée par le fait que** la phase grasse liquide comprend une huile choisie dans le groupe formé par les huiles d'origine minérale, animale, végétale ou synthétique, hydrocarbonées, fluorées et/ou siliconées, seules ou en mélange.

31. Composition selon la revendication 29 ou 30, **caractérisée par le fait qu'**elle comprend une huile volatile à température ambiante.

32. Composition selon l'une quelconque des revendications 29 à 31, **caractérisée par le fait qu'**elle comprend une huile volatile choisie parmi les huiles volatiles hydrocarbonées ayant de 8 à 16 atomes de carbone.

33. Composition selon la revendication 31 ou 32, **caractérisée par le fait que** l'huile volatile est présente en une teneur allant de de 0,1 % à 98 % en poids, par rapport au poids total de la composition, de préférence allant de 1 % à 65 %.

34. Composition selon l'une quelconque des revendications 29 à 33, **caractérisée par le fait que** la phase grasse liquide est présente en une teneur allant de 2 % à 98 % en poids, par rapport au poids total de la composition, de préférence allant de 5 % à 85 % en poids.

35. Composition selon l'une quelconque des revendications 1 à 34, **caractérisée par le fait que** le deuxième polymère filmogène est présent sous la forme de particules dispersées dans une phase grasse liquide et stabilisées en surface.

36. Composition selon la revendication 35, **caractérisée par le fait que** les particules de polymères sont stabilisées par un stabilisant choisi parmi les polymères séquencés, les polymères greffés, les polymères statistiques et leurs mélanges.

37. Composition selon la revendication 36, **caractérisée par le fait que** le stabilisant est un polymère bloc greffé ou séquencé, comprenant au moins un bloc résultant de la polymérisation de monomères éthyléniques comportant une ou plusieurs liaisons éthylénique éventuellement conjuguées et au moins un bloc d'un polymère styrénique.

38. Composition selon l'une quelconque des revendications précédentes, **caractérisée par le fait que** le deuxième polymère filmogène est présent en une teneur allant de 0,1 % à 60 % en poids, par rapport au poids total de la composition, de préférence de 0,5 % à 40 % en poids.

39. Composition selon l'une quelconque des revendications précédentes, **caractérisée par le fait que** la taille des particules du deuxième polymère filmogène va de 5 nm à 600 nm, et de préférence de 20 nm à 300 nm.

40. Composition selon l'une quelconque des revendications précédentes, **caractérisée par le fait qu'**elle contient au moins une cire.

41. Composition selon la revendication 40, **caractérisée par le fait qu'**elle contient au moins une cire ayant une température de fusion supérieure à 30°C et allant jusqu'à 120 °C.

42. Composition selon la revendication 40 ou 41, **caractérisée par le fait qu'**elle contient une cire choisie dans le groupe formé par la cire d'abeilles, la cire de lanoline, les cires d'insectes de Chine, la cire de riz, la cire de Carnauba, la cire de Candellila, la cire d'Ouricury, la cire de fibres de liège, la cire de canne à sucre, la cire du Japon et la cire de sumac, la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C, les cires obtenues par hydrogénation catalytique d'huiles animales ou végétales ayant des chaînes grasses, linéaires ou ramifiées, en C8-C32, les cires de silicone, les cires fluorées, et leurs mélanges.

43. Composition selon l'une quelconque des revendications 40 à 42, **caractérisée par le fait que** la cire est présente en une teneur allant de 0,1 % à 50 % en poids, par rapport au poids total de la composition, de préférence de 0,5 % à 30 % en poids, et mieux de 1 % à 20 % en poids.

44. Composition selon l'une des revendications précédentes, **caractérisée par le fait qu'**elle contient, en outre, au moins une matière colorante.

45. Composition selon la revendication 44, **caractérisée par le fait que** la matière colorante est choisie parmi les pigments, les nacres, les colorants liposolubles, les colorants hydrosolubles, et leurs mélanges.

46. Composition selon la revendication 44 ou 45, **caractérisée par le fait que** la matière colorante est présente à raison de 0,01 à 30 % du poids total de la composition.

47. Composition selon l'une des revendications précédentes, **caractérisée par le fait qu'**elle constitue une composition de soin ou de maquillage des matières kératiniques.

48. Composition selon l'une des revendications précédentes, **caractérisée par le fait qu'**elle contient au moins un additif choisi parmi les antioxydants, les charges, les conservateurs, les parfums, les neutralisants, les épaississants, les actifs cosmétiques ou dermatologiques, et leurs mélanges.

49. Composition selon l'une des revendications précédentes, **caractérisée en ce qu'**elle se présente sous forme de mascara, d'eye-liner, de produit pour les sourcils, de produit pour les lèvres, de fard à joues ou à paupières, de fond de teint, de produit de maquillage du corps, de produit anti-cernes, de vernis à ongles, de produit de soin de la peau, de produit pour les cheveux.

50. Mascara comprenant une composition selon l'une quelconque des revendications 1 à 49.

51. Procédé cosmétique de maquillage ou de soin des matières kératiniques des êtres humains, comprenant l'application sur les matières kératiniques d'une composition cosmétique conforme à l'une des revendications 1 à 49.

52. Utilisation d'une composition selon l'une quelconque des revendications 1 à 49 pour l'obtention d'un dépôt adhérent sur les matières kératiniques et/ou un maquillage rapide des matières kératiniques.

53. Utilisation d'un mascara selon la revendication 52 pour épaissir rapidement et/ou allonger les cils.

54. Utilisation, dans une composition physiologiquement acceptable, de l'association d'au moins un premier polymère de masse moléculaire moyenne en poids inférieure à 100 000 et mieux inférieure à 50 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés, et d'au moins une dispersion de particules un deuxième polymère filmogène insoluble dans ledit milieu, pour l'obtention d'un dépôt adhérent sur les matières kératiniques et/ou d'un maquillage rapide des matières kératiniques et/ou pour épaissir rapidement et/ou allonger les cils.

55. Utilisation selon la revendication 54, **caractérisée par le fait que** le polymère est un polyamide comportant des groupements terminaux à groupe ester comportant une chaîne hydrocarbonée ayant de 10 à 42 atomes de carbone.

56. Utilisation selon la revendication 54 ou 55, **caractérisée par le fait que** le premier polymère a une masse moléculaire moyenne en poids allant de 1 000 à 30 000.

## Claims

1. Composition comprising, in a physiologically acceptable medium, at least one first polymer with a weight-average molecular mass of less than 100 000, comprising a) a polymer backbone containing hydrocarbon-based repeating units containing at least one heteroatom, and optionally b) at least one pendent fatty chain and/or at least one terminal fatty chain, which may be functionalized, containing from 6 to 120 carbon atoms and being linked to these hydrocarbon-based units, and a dispersion of particles of a second film-forming polymer that is insoluble in said medium.

2. Composition according to Claim 1, **characterized in that** the average molar mass of the first polymer is less than 50 000.

3. Composition according to Claim 1 or 2, **characterized in that** the units containing a heteroatom of the first polymer comprise a nitrogen atom.

4. Composition according to one of the preceding claims, **characterized in that** the units containing a heteroatom of the first polymer are amide groups.

5. Composition according to one of the preceding claims, **characterized in that** the fatty chains represent from 40% to 98% and better still from 50% to 95% of the total number of units containing a heteroatom and of fatty chains.

6. Composition according to one of the preceding claims, **characterized in that** the pendent fatty chains are linked directly to at least one of said heteroatoms.

7. Composition comprising, in a physiologically acceptable medium, at least one first polyamide polymer with a weight-average molecular mass of less than 100 000, comprising a) a polymer backbone containing amide repeating units, and b) optionally at least one pendent fatty chain and/or at least one terminal fatty chain, which may be functionalized, containing from 8 to 120 carbon atoms and being linked to these amide units, and a dispersion of particles of a second film-forming polymer that is insoluble in said medium.

8. Composition according to the preceding claim, **characterized in that** the fatty chains represent from 40% to 98% of the total number of amide units and of fatty chains.

9. Composition according to Claim 7 or 8, **characterized in that** the fatty chains represent from 50% to 95% of the total number of amide units and of fatty chains.

10. Composition according to one of Claims 7 to 10, **characterized in that** the pendent fatty chains are linked directly to at least one of the nitrogen atoms of the amide units.

11. Composition according to one of the preceding claims, **characterized in that** the average molar mass of the first polymer ranges from 1 000 to 100 000, preferably from 1 000 to 50 000 and better still from 1 000 to 30 000.

12. Composition according to one of the preceding claims, **characterized in that** the weight-average molar mass of the first film-forming polymer ranges from 2 000 to 20 000 and preferably from 2 000 to 10 000.

13. Composition according to one of the preceding claims, **characterized in that** the terminal fatty chain(s) is (are) linked to the backbone via bonding groups.

14. Composition according to Claim 13, **characterized in that** the bonding groups are ester groups.

15. Composition according to one of the preceding claims, **characterized in that** the fatty chain(s) contain(s) from 12 to 68 carbon atoms.

16. Composition according to one of the preceding claims, **characterized in that** the first polymer is chosen from polymers of formula (I) below, and mixtures thereof: 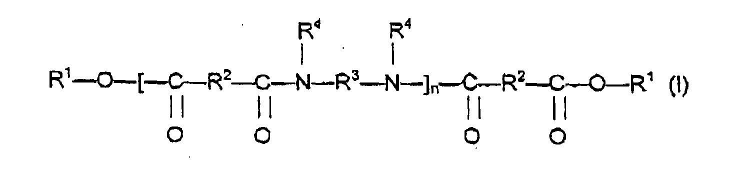 in which n denotes a number of amide units such that the number of ester groups represents from 10% to 50% of the total number of ester and amide groups; R¹ is, independently in each case, an alkyl or alkenyl group containing at least 4 carbon atoms; R² represents, independently in each case, a C₄ to C₄₂ hydrocarbon-based group, on condition that 50% of the groups R² represent a C₃₀ to C₄₂ hydrocarbon-based group; R³ represents, independently in each case, an organic group containing at least 2 carbon atoms, hydrogen atoms and optionally one or more oxygen or nitrogen atoms; and R⁴ represents, independently in each case, a hydrogen atom, a C₁ to C₁₀ alkyl group or a direct bond to R³ or to another R⁴, such that the nitrogen atom to which R³ and R⁴ are both attached forms part of a heterocyclic structure defined by R⁴-N-R³, with at least 50% of the groups R⁴ representing a hydrogen atom.

17. Composition according to the preceding claim, **characterized in that** R¹ is a C₁₂ to C₂₂ alkyl group.

18. Composition according to either of Claims 15 and 16, **characterized in that** R² are groups containing from 30 to 42 carbon atoms.

19. Composition according to one of the preceding claims, **characterized in that** the first polymer is present in a content ranging from 0.01% to 10% by weight, relative to the total weight of the composition, preferably ranging from 0.05% to 5% by weight and better still ranging from 0.1% to 3% by weight.

20. Composition according to any one of the preceding claims, **characterized in that** the second film-forming polymer is chosen from the group formed by free-radical polymers, polycondensates and polymers of natural origin, and blends thereof.

21. Composition according to any one of the preceding claims, **characterized in that** the second film-forming polymer is chosen from the group formed by vinyl polymers, polyurethanes, polyesters and cellulose polymers.

22. Composition according to any one of the preceding claims, **characterized in that** it comprises an aqueous phase.

23. Composition according to Claim 22, **characterized in that** the aqueous phase comprises water and optionally a water-miscible organic solvent.

24. Composition according to Claim 23, **characterized in that** the water-miscible organic solvent is chosen from the group formed by lower monoalcohols containing from 1 to 5 carbon atoms, glycols containing from 2 to 8 carbon atoms, C₃-C₄ ketones and C₂-C₄ aldehydes.

25. Composition according to Claim 23 or 24, **characterized in that** the water-miscible organic solvent is chosen from the group formed by ethanol, isopropanol, propylene glycol, ethylene glycol, 1,3-butylene glycol and dipropylene glycol.

26. Composition according to any one of Claims 23 to 25, **characterized in that** it comprises water in a content ranging from 1% to 95% by weight relative to the total weight of the composition.

27. Composition according to any one of the preceding claims, **characterized in that** the second film-forming polymer is present in the form of particles dispersed in an aqueous phase.

28. Composition according to any one of Claims 22 to 27, **characterized in that** the aqueous phase comprises an additional water-soluble film-forming polymer.

29. Composition according to any one of the preceding claims, **characterized in that** it comprises a liquid fatty phase.

30. Composition according to Claim 29, **characterized in that** the liquid fatty phase comprises an oil chosen from the group formed by oils of mineral, animal, plant or synthetic origin, that are hydrocarbon-based, fluorinated and/or silicone-based, alone or as a mixture.

31. Composition according to Claim 29 or 30, **characterized in that** it comprises an oil that is volatile at room temperature.

32. Composition according to any one of Claims 29 to 31, **characterized in that** it comprises a volatile oil chosen from hydrocarbon-based volatile oils containing from 8 to 16 carbon atoms.

33. Composition according to Claim 31 or 32, **characterized in that** the volatile oil is present in a content ranging from 0.1% to 98% and preferably ranging from 1% to 65% by weight, relative to the total weight of the composition.

34. Composition according to any one of Claims 29 to 33, **characterized in that** the liquid fatty phase is present in a content ranging from 2% to 98% by weight and preferably ranging from 5% to 85% by weight, relative to the total weight of the composition.

35. Composition according to any one of Claims 1 to 34, **characterized in that** the second film-forming polymer is present in the form of particles dispersed in a liquid fatty phase and surface-stabilized.

36. Composition according to Claim 35, **characterized in that** the polymer particles are stabilized with a stabilizer chosen from block polymers, grafted polymers and random polymers, and blends thereof.

37. Composition according to Claim 36, **characterized in that** the stabilizer is a grafted-block or block polymer, comprising at least one block resulting from the polymerization of ethylenic monomers comprising one or more optionally conjugated ethylenic bonds, and at least one block of a styrene polymer.

38. Composition according to any one of the preceding claims, **characterized in that** the second film-forming polymer is present in a content ranging from 0.1% to 60% by weight and preferably from 0.5% to 40% by weight, relative to the total weight of the composition.

39. Composition according to any one of the preceding claims, **characterized in that** the size of the particles of the second film-forming polymer ranges from 5 nm to 600 nm and preferably from 20 nm to 300 nm.

40. Composition according to any one of the preceding claims, **characterized in that** it contains at least one wax.

41. Composition according to Claim 40, **characterized in that** it contains at least one wax having a melting point of greater than 30°C, which may be up to 120°C.

42. Composition according to Claim 40 or 41, **characterized in that** it contains a wax chosen from the group formed by beeswax, lanolin wax, Chinese insect waxes, rice wax, carnauba wax, candelilla wax, ouricury wax, cork fiber wax, sugar cane wax, Japan wax, sumach wax, montan wax, microcrystalline waxes, paraffin waxes, ozokerites, ceresin wax, lignite wax, polyethylene waxes and the waxes obtained by Fisher-Tropsch synthesis, fatty acid esters of glycerides that are solid at 40°C, the waxes obtained by catalytic hydrogenation of animal or plant oils containing linear or branched C₈-C₃₂ fatty chains, silicone waxes and fluoro waxes, and mixtures thereof.

43. Composition according to any one of Claims 40 to 42, **characterized in that** the wax is present in a content ranging from 0.1% to 50% by weight, relative to the total weight of the composition, preferably from 0.5% to 30% by weight and better still from 1% to 20% by weight.

44. Composition according to one of the preceding claims, **characterized in that** it also contains at least one dyestuff.

45. Composition according to Claim 44, **characterized in that** the dyestuff is chosen from pigments, nacres, liposoluble dyes and water-soluble dyes, and mixtures thereof.

46. Composition according to Claim 44 or 45, **characterized in that** the dyestuff is present in a proportion of from 0.01% to 30% relative to the total weight of the composition.

47. Composition according to one of the preceding claims, **characterized in that** it constitutes a care composition or makeup composition for keratin materials.

48. Composition according to one of the preceding claims, **characterized in that** it contains at least one additive chosen from antioxidants, fillers, preserving agents, fragrances, neutralizers, thickeners and cosmetic or dermatological active agents, and mixtures thereof.

49. Composition according to one of the preceding claims, **characterized in that** it is in the form of a mascara, an eyeliner, a product for the eyebrows, a product for the lips, a face powder, an eyeshadow, a foundation, a makeup product for the body, a concealer product, a nail varnish, a skincare product or a haircare product.

50. Mascara comprising a composition according to any one of Claims 1 to 49.

51. Cosmetic process for making up or caring for the keratin materials of human beings, comprising the application of a cosmetic composition in accordance with one of Claims 1 to 49 to the keratin materials.

52. Use of a composition according to any one of Claims 1 to 49 to obtain a deposit which adheres to keratin materials and/or a quick makeup result on keratin materials.

53. Use of a mascara according to Claim 52 to thicken quickly and/or to lengthen the eyelashes.

54. Use, in a physiologically acceptable composition, of a combination of at least one first polymer with a weight-average molecular mass of less than 100 000 and better still less than 50 000, comprising a) a polymer backbone containing hydrocarbon-based repeating units containing at least one heteroatom, and b) optionally at least one pendent fatty chain and/or at least one terminal fatty chain, which may be functionalized, containing from 6 to 120 carbon atoms and being linked to these hydrocarbon-based units, and at least one dispersion of particles of a second film-forming polymer that is insoluble in said medium, to obtain a deposit which adheres to keratin materials and/or a quick makeup for keratin materials and/or to thicken quickly and/or lengthen the eyelashes.

55. Use according to Claim 54, **characterized in that** the polymer is a polyamide comprising end groups containing an ester group comprising a hydrocarbon-based chain containing from 10 to 42 carbon atoms.

56. Use according to Claim 54 or 55, **characterized in that** the first polymer has a weight-average molecular mass ranging from 1 000 to 30 000.

## Patentansprüche

1. Zusammensetzung, die in einem physiologisch akzeptablen Medium mindestens ein erstes Polymer mit einer gewichtsmittleren Molmasse unter 100 000, das a) ein Polymergrundgerüst mit Wiederholungseinheiten auf Kohlenwasserstoffbasis, die mit mindestens einem Heteroatom versehen sind, und gegebenenfalls b) mindestens eine als Seitenkette vorliegende Fettkette und/oder mindestens eine endständige Fettkette aufweist, die gegebenenfalls funktionalisiert sind, 6 bis 120 Kohlenstoffatome aufweisen und an die Kohlenwasserstoffeinheiten gebunden sind, und eine Dispersion von Partikeln eines zweiten, in dem Medium unlöslichen, filmbildenden Polymers enthält.

2. Zusammensetzung nach Anspruch 1, **dadurch gekennzeichnet, dass** die mittlere Molmasse des ersten Polymers unter 50 000 liegt.

3. Zusammensetzung nach Anspruch 1 oder 2, **dadurch gekennzeichnet, dass** die Einheiten des ersten Polymers mit Heteroatom ein Stickstoffatom enthalten.

4. Zusammensetzung nach einem der vorhergehenden Ansprüche, **dadurch gekennzeichnet, dass** die Einheiten des ersten Polymers mit Heteroatom Amidgruppen sind.

5. Zusammensetzung nach einem der vorhergehenden Ansprüche, **dadurch gekennzeichnet, dass** die Fettketten 40 bis 98 % und besser 50 bis 95 % der Gesamtzahl der Einheiten mit Heteroatom und der Fettketten ausmachen.

6. Zusammensetzung nach einem der vorhergehenden Ansprüche, **dadurch gekennzeichnet, dass** die als Seitenketten vorliegenden Fettketten zumindest an eines der Heteroatome direkt gebunden sind.

7. Zusammensetzung, die in einem physiologisch akzeptablen Medium mindestens ein erstes Polyamid-Polymer mit einer gewichtsmittleren Molmasse unter 100 000, das a) ein Polymergrundgerüst mit wiederkehrenden Amideinheiten und gegebenenfalls b) mindestens eine als Seitenkette vorliegende Fettkette und/oder mindestens eine endständige Fettkette aufweist, die gegebenenfalls funktionalisiert sind, 8 bis 120 Kohlenstoffatome aufweisen und an die Amideinheiten gebunden sind, und eine Dispersion von Partikeln eines zweiten, in dem Medium unlöslichen, filmbildenden Polymers enthält.

8. Zusammensetzung nach dem vorhergehenden Anspruch, **dadurch gekennzeichnet, dass** die Fettketten 40 bis 98 % der Gesamtzahl der Amideinheiten und Fettketten ausmachen.

9. Zusammensetzung nach Anspruch 7 oder 8, **dadurch gekennzeichnet, dass** die Fettketten 50 bis 95 % der Anzahl der Amideinheiten und Fettketten ausmachen.

10. Zusammensetzung nach einem der Ansprüche 7 bis 10, **dadurch gekennzeichnet, dass** die als Seitenketten vorliegenden Fettketten an zumindest eines der Stickstoffatome der Amideinheiten direkt gebunden sind.

11. Zusammensetzung nach einem der vorhergehenden Ansprüche, **dadurch gekennzeichnet, dass** die mittlere Molmasse des ersten Polymers im Bereich von 1 000 bis 100 000, vorzugsweise 1 000 bis 50 000 und besser 1 000 bis 30 000 liegt.

12. Zusammensetzung nach einem der vorhergehenden Ansprüche, **dadurch gekennzeichnet, dass** die gewichtsmittlere Molmasse des ersten filmbildenden Polymers im Bereich von 2 000 bis 20 000 und vorzugsweise 2 000 bis 10 000 liegt.

13. Zusammensetzung nach einem der vorhergehenden Ansprüche, **dadurch gekennzeichnet, dass** die endständige(n) Fettkette(n) an das Grundgerüst über Verbindungsgruppen gebunden sind.

14. Zusammensetzung nach Anspruch 13, **dadurch gekennzeichnet, dass** die Verbindungsgruppen Estergruppen sind.

15. Zusammensetzung nach einem der vorhergehenden Ansprüche, **dadurch gekennzeichnet, dass** die Fettkette(n) 12 bis 68 Kohlenstoffatome aufweisen.

16. Zusammensetzung nach einem der vorhergehenden Ansprüche, **dadurch gekennzeichnet, dass** das erste Polymer unter den Polymeren der folgenden Formel (I) und deren Gemischen ausgewählt ist: 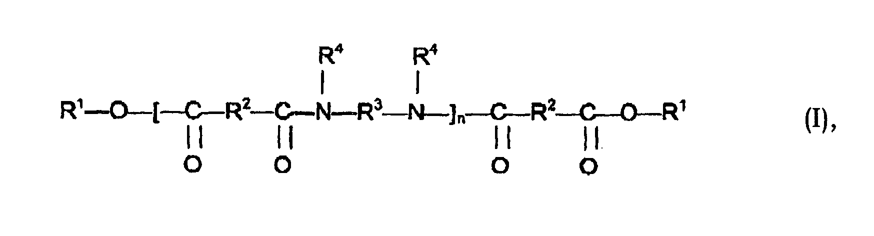 worin n die Anzahl der Amideinheiten angibt, die so ist, dass die Zahl der Estergruppen 10 bis 50 % der Gesamtzahl der Ester-und Amidgruppen ausmacht; die Gruppen R¹ jeweils unabhängig eine Alkyl- oder Alkenylgruppe mit mindestens 4 Kohlenstoffatomen bedeutet, die Gruppen R² jeweils unabhängig eine Kohlenwasserstoffgruppe mit 4 bis 42 Kohlenstoffatomen ist, mit der Maßgabe, dass 50 % der Gruppen R² eine Kohlenwasserstoffgruppe mit 30 bis 42 Kohlenstoffatomen bedeutet; die Gruppen R³ jeweils unabhängig eine organische Gruppe, die mindestens 2 Kohlenstoffatome, Wasserstoffatome und optional ein oder mehrere Sauerstoff- oder Stickstoffatome aufweist; und die Gruppen R⁴ jeweils unabhängig ein Wasserstoffatom, C₁₋₁₀-Alkyl oder eine direkte Bindung zu R³ oder einer weiteren Gruppe R⁴ bedeuten, sodass das Stickstoffatom, an das die Gruppen R³ und R⁴ gebunden sind, Teil einer durch R⁴-N-R³ definierten heterocyclischen Struktur ist, wobei mindestens 50 % der Gruppen R⁴ ein Wasserstoffatom bedeuten.

17. Zusammensetzung nach dem vorhergehenden Anspruch, **dadurch gekennzeichnet, dass** R¹ eine Alkylgruppe mit 12 bis 22 Kohlenstoffatomen ist.

18. Zusammensetzung nach einem der Ansprüche 15 oder 16, **dadurch gekennzeichnet, dass** die Gruppen R² Gruppen mit 30 bis 42 Kohlenstoffatomen sind.

19. Zusammensetzung nach einem der vorhergehenden Ansprüche, **dadurch gekennzeichnet, dass** das erste Polymer in einer Menge von 0,01 bis 10 Gew.-%, bezogen auf das Gesamtgewicht der Zusammensetzung, vorzugsweise 0,05 bis 5 Gew.-% und besser 0,1 bis 3 Gew.-% enthalten ist.

20. Zusammensetzung nach einem der vorhergehenden Ansprüche, **dadurch gekennzeichnet, dass** das zweite filmbildende Polymer unter den durch radikalische Polymerisation hergestellten Polymeren, Polykondensaten, Polymeren natürlicher Herkunft und deren Gemischen ausgewählt ist.

21. Zusammensetzung nach einem der vorhergehenden Ansprüche, **dadurch gekennzeichnet, dass** das zweite filmbildende Polymer unter den Vinylpolymeren, Polyurethanen, Polyestern, Cellulosepolymeren ausgewählt ist.

22. Zusammensetzung nach einem der vorhergehenden Ansprüche, **dadurch gekennzeichnet, dass** sie eine wässrige Phase aufweist.

23. Zusammensetzung nach Anspruch 22, **dadurch gekennzeichnet, dass** die wässrige Phase Wasser und gegebenenfalls ein mit Wasser mischbares organisches Lösungsmittel enthält.

24. Zusammensetzung nach Anspruch 23, **dadurch gekennzeichnet, dass** das mit Wasser mischbare organische Lösungsmittel unter den niederen Monoalkoholen mit 1 bis 5 Kohlenstoffatomen, Glycolen mit 2 bis 8 Kohlenstoffatomen, C₃₋₄-Ketonen und C₂₋₄-Aldehyden ausgewählt ist.

25. Zusammensetzung nach Anspruch 23 oder 24, **dadurch gekennzeichnet, dass** das mit Wasser mischbare organische Lösungsmittel unter Ethanol, Isopropanol, Propylenglycol, Ethylenglycol, 1,3-Butylenglycol und Dipropylenglycol ausgewählt ist.

26. Zusammensetzung nach einem der Ansprüche 23 bis 25, **dadurch gekennzeichnet, dass** sie das Wasser in einer Menge von 1 bis 95 Gew.-%, bezogen auf das Gesamtgewicht der Zusammensetzung, enthält.

27. Zusammensetzung nach einem der vorhergehenden Ansprüche, **dadurch gekennzeichnet, dass** das zweite filmbildende Polymer in Form von in einer wässrigen Phase dispergierten Partikeln vorliegt.

28. Zusammensetzung nach einem der Ansprüche 22 bis 27, **dadurch gekennzeichnet, dass** die wässrige Phase ein ergänzendes wasserlösliches filmbildendes Polymer enthält.

29. Zusammensetzung nach einem der vorhergehenden Ansprüche, **dadurch gekennzeichnet, dass** sie eine flüssige Fettphase enthält.

30. Zusammensetzung nach Anspruch 29, **dadurch gekennzeichnet, dass** die flüssige Fettphase ein Öl enthält, das unter den Ölen mineralischer, tierischer, pflanzlicher oder synthetischer Herkunft, Kohlenwasserstoffölen, fluorierten Ölen und/oder Siliconölen oder deren Gemischen ausgewählt ist.

31. Zusammensetzung nach einem der Ansprüche 29 oder 30, **dadurch gekennzeichnet, dass** sie ein bei Raumtemperatur flüchtiges Öl enthält.

32. Zusammensetzung nach einem der Ansprüche 29 bis 31, **dadurch gekennzeichnet, dass** sie ein flüchtiges Öl enthält, das unter den flüchtigen Kohlenwasserstoffölen mit 8 bis 16 Kohlenstoffatomen ausgewählt ist.

33. Zusammensetzung nach Anspruch 31 oder 32, **dadurch gekennzeichnet, dass** das flüchtige Öl in einer Menge von 0,1 bis 98 Gew.-%, bezogen auf das Gesamtgewicht der Zusammensetzung, und vorzugsweise 1 bis 65 % vorliegt.

34. Zusammensetzung nach einem der Ansprüche 29 bis 33, **dadurch gekennzeichnet, dass** die flüssige Fettphase in einer Menge von 2 bis 98 Gew.-%, bezogen auf das Gesamtgewicht der Zusammensetzung, und vorzugsweise 5 bis 85 Gew.-% vorliegt.

35. Zusammensetzung nach einem der Ansprüche 1 bis 34, **dadurch gekennzeichnet, dass** das zweite filmbildende Polymer in Form von in einer flüssigen Fettphase dispergierten und an der Oberfläche stabilisierten Partikeln vorliegt.

36. Zusammensetzung nach Anspruch 35, **dadurch gekennzeichnet, dass** die Polymerpartikel durch ein Stabilisierungsmittel stabilisiert sind, das unter den Sequenzpolymeren, Pfropfpolymeren, statistischen Polymeren und deren Gemischen ausgewählt ist.

37. Zusammensetzung nach Anspruch 36, **dadurch gekennzeichnet, dass** das Stabilisierungsmittel ein gepfropftes oder sequentielles Blockpolymer ist, das mindestens einen Block, der bei der Polymerisation von ethylenischen Monomeren mit einer oder mehreren, gegebenenfalls konjugierten Doppelbindungen gebildet wird, und mindestens einen Block eines Styrolpolymers aufweist.

38. Zusammensetzung nach einem der vorhergehenden Ansprüche, **dadurch gekennzeichnet, dass** das zweite filmbildende Polymer in einer Menge von 0,1 bis 60 Gew.-%, bezogen auf das Gesamtgewicht der Zusammensetzung, und vorzugsweise 0,5 bis 40 Gew.-% vorliegt.

39. Zusammensetzung nach einem der vorhergehenden Ansprüche, **dadurch gekennzeichnet, dass** die Partikelgröße des zweiten filmbildenden Polymers im Bereich von 5 bis 600 nm und vorzugsweise 20 bis 300 nm liegt.

40. Zusammensetzung nach einem der vorhergehenden Ansprüche, **dadurch gekennzeichnet, dass** sie mindestens ein Wachs enthält.

41. Zusammensetzung nach Anspruch 40, **dadurch gekennzeichnet, dass** sie mindestens ein Wachs mit einer Schmelztemperatur über 30 °C und bis zu 120 °C enthält.

42. Zusammensetzung nach Anspruch 40 oder 41, **dadurch gekennzeichnet, dass** sie ein Wachs enthält, das unter den Bienenwachsen, Lanolinwachs, Chinawachsen, Reiswachs, Carnaubawachs, Candelillawachs, Ouricurywachs, Korkfaserwachs, Zuckerrohrwachs, Japanwachs und Sumachwachs, Montanwachs, mikrokristallinen Wachsen, Paraffinwachsen, Ozokeriten, Ceresinwachs, Lignit, Polyethylenwachsen, durch Fischer-Tropsch-Synthese hergestellten Wachsen, Estern von Fettsäuren und Glyceriden, die bei 40 °C fest sind, durch katalytische Hydrierung von tierischen oder pflanzlichen Ölen mit geradkettigen oder verzweigten C₈₋₃₂-Fettketten erhaltenen Wachsen, Siliconwachsen, fluorierten Wachsen und deren Gemischen ausgewählt ist.

43. Zusammensetzung nach einem der Ansprüche 40 bis 42, **dadurch gekennzeichnet, dass** das Wachs in einer Menge von 0,1 bis 50 Gew.-%, bezogen auf das Gesamtgewicht der Zusammensetzung, vorzugsweise 0,5 bis 30 Gew.-% und besser 1 bis 20 Gew.-% enthalten ist.

44. Zusammensetzung nach einem der vorhergehenden Ansprüche, **dadurch gekennzeichnet, dass** sie ferner mindestens ein Farbmittel enthält.

45. Zusammensetzung nach Anspruch 44, **dadurch gekennzeichnet, dass** das Farbmittel unter den Pigmenten, Perlglanzpigmenten, fettlöslichen Farbstoffen, wasserlöslichen Farbstoffen und deren Gemischen ausgewählt ist.

46. Zusammensetzung nach Anspruch 44 oder 45, **dadurch gekennzeichnet, dass** das Farbmittel in einer Menge von 0,01 bis 30 % des Gesamtgewichts der Zusammensetzung vorliegt.

47. Zusammensetzung nach einem der vorhergehenden Ansprüche, **dadurch gekennzeichnet, dass** sie eine Zusammensetzung für die Pflege oder zum Schminken von Keratinsubstanzen ist.

48. Zusammensetzung nach einem der vorhergehenden Ansprüche, **dadurch gekennzeichnet, dass** sie mindestens einen Zusatzstoff enthält, der unter den Antioxidantien, Füllstoffen, Konservierungsmitteln, Parfums, Neutralisationsmitteln, Verdickungsmitteln, kosmetischen oder dermatologischen Wirkstoffen und deren Gemischen ausgewählt ist.

49. Zusammensetzung nach einem der vorhergehenden Ansprüche, **dadurch gekennzeichnet, dass** sie als Mascara, Eyeliner, Produkt für die Augenbrauen, Produkt für die Lippen, Lidschatten oder Wangenrouge, Make-up, Produkt zum Schminken des Körpers, Produkt gegen Augenringe, Nagellack, Produkt für die Pflege der Haut und Produkt für die Haare vorliegt.

50. Mascara, die eine Zusammensetzung nach einem der Ansprüche 1 bis 49 enthält.

51. Kosmetisches Verfahren zum Schminken oder für die Pflege von menschlichen Keratinsubstanzen, das das Aufbringen einer kosmetischen Zusammensetzung nach einem der Ansprüche 1 bis 49 auf die Keratinsubstanzen umfasst.

52. Verwendung einer Zusammensetzung nach einem der Ansprüche 1 bis 49 für die Bildung einer haftenden Ablagerung auf den Keratinsubstanzen und/oder zum schnellen Schminken von Keratinsubstanzen.

53. Verwendung einer Mascara nach Anspruch 52, um die Wimpern schnell zu verdicken und/oder zu verlängern.

54. Verwendung der Kombination aus mindestens einem ersten Polymer mit einer gewichtsmittleren Molmasse unter 100 000 und besser unter 50 000, das a) ein Polymergrundgerüst mit Wiederholungseinheiten auf Kohlenwasserstoffbasis, die mit mindestens einem Heteroatom versehen sind, und b) gegebenenfalls mindestens eine als Seitenkette vorliegende Fettkette und/oder mindestens eine endständige Fettkette aufweist, welche gegebenenfalls funktionalisiert sind, 6 bis 120 Kohlenstoffatome aufweisen und an die Kohlenwasserstoffeinheiten gebunden sind, und mindestens einer Dispersion von Partikeln eines in dem Medium unlöslichen, filmbildenden zweiten Polymers in einer physiologisch akzeptablen Zusammensetzung für die Bildung einer haftenden Ablagerung auf den Keratinsubstanzen und/oder zum schnellen Schminken von Keratinsubstanzen und/oder zur schnellen Verdickung und/oder Verlängerung der Wimpern.

55. Verwendung nach Anspruch 54, **dadurch gekennzeichnet, dass** das Polymer ein Polyamid ist, das endständige Gruppen mit Estergruppe aufweist, die eine Kohlenwasserstoffkette mit 10 bis 42 Kohlenstoffatomen enthalten.

56. Verwendung nach Anspruch 54 oder 55, **dadurch gekennzeichnet, dass** das erste Polymer eine gewichtsmittlere Molmasse von 1 000 bis 30 000 aufweist.
